# EUROPEAN PATENT APPLICATION

(11) **EP 2 412 386 A1**
(43) Date of publication of application: **01.02.2012**
(21) Application number: 10756117.7
(22) Date of filing: 24.03.2010
(51) Int. Cl.: A61K 47/38, A61K 9/24, A61K 9/70, A61K 47/32

(54) **ADHESION PREVENTING COMPOSITION, SOLID PREPARATION AND METHOD FOR PRODUCING SAME**

(30) Priority: 25.03.2009 JP 2009074160
(71) Applicant: LINTEC Corporation, Tokyo 173-0001 (JP); ASKA Pharmaceutical Co., Ltd., Minato-ku, Tokyo 108-8532 (JP)
(72) Inventor: SUGIURA Yusaku, Tokyo 173-0001 (JP); TAKANO Youichi, Tokyo 173-0001 (JP)
(74) Representative: von Kreisler Selting Werner
(86) International application number: PCT/JP2010/055093
(87) International publication number: WO 2010/110321

(57) **Abstract**

An adhesion of a preparation 1 to an inner wall of an oral cavity is prevented by applying an anti-adhesive composition containing a water-soluble cellulose ether (e.g., an HPMC) and an anionic polymer (e.g., a carboxyvinyl polymer) to a gel-forming layer 4 covering a drug-containing unit 2 to form an anti-adhesive layer 5. The gel-forming layer 4 maybe formed with a gel-forming agent (a carboxyvinyl polymer), a crosslinking agent, and a base material (e.g., a poly(vinyl alcohol)). An adhesive layer 3 may be interposed between the drug-containing unit 2 and the gel-forming layer 4.

## Description

### TECHNICAL FIELD

The present invention relates to an anti-adhesive composition (or a coating agent) for preventing a preparation from adhering to an inner wall of the oral cavity, a solid preparation (for example, a solid preparation for oral administration, such as a film-covered preparation) covered with the anti-adhesive composition (or a solid preparation having a covering layer comprising the anti-adhesive composition), and a process for producing the preparation.

### BACKGROUND ART

As a conventional dosage form for an oral administration preparation, a solid preparation (e.g., tablets, granules, and powders) is used. Moreover, the solid preparation is covered (or coated) according to various purposes.

For imparting to a luster (or a gloss) to an external appearance of a solid preparation, Japanese Patent Application Laid-Open NO. 2002-534373 (JP-2002-534373A, Patent Document 1) discloses a coating composition providing high gloss coatings containing a hydroxypropyl cellulose and an anionic polymer. This document exemplifies the anionic polymer, a sodium carboxymethyl cellulose, a carboxyvinyl polymer, and others, and discloses that the composition containing the hydroxypropyl cellulose and the carboxymethyl cellulose in a mass ratio of the hydroxypropyl cellulose relative to the carboxymethyl cellulose of about 1/20 to about 20/1 is suitable for a coating (for example, a coating for tablets, granules, and the like). However, the solid preparation is difficult to swallow with a small quantity of water or moisture (e.g., saliva) because of a size thereof or a bitterness or acerbity of a drug contained therein. Moreover, depending on the shape of the preparation after coating, the adherability of the preparation to an inner wall of the oral cavity increases, the comfortability (or feeling or acceptability) of taking the preparation is lowered, and a large quantity of water is required for taking the preparation. When taken in such a manner, the solid preparation has a risk of blocking the respiratory tract by accident.

WO 02/087622 (Patent Document 2) discloses a solid preparation for oral administration, in which a drug-containing layer is covered with a water-swellable gel-forming layer, and also discloses that the water-swellable gel-forming layer may contain a film-forming agent such as a poly(vinyl alcohol) or a hydroxyalkyl cellulose. This document also exemplifies compositions containing a poly(vinyl alcohol) (film-forming agent), a polyacrylic acid (a water-swellable gel-forming agent), and calcium chloride in ratios of 100/0/0 to 85/15/0 (% by mass), 95/4.5/0.5 (% by mass), and 85/13.5/1.5 (% by mass), and describes that these compositions are insufficient as a composition for forming a water-swellable gel-forming layer. Since the solid preparation described in Patent Document 2 forms a gel layer by absorbing waterinthewater-swellablegel-forminglayer, the preparation can be taken with a small quantity of water. However, even this solid preparation sometimes adheres to an inner wall of the oral cavity because of water in the oral cavity, so that the solid preparation cannot be swallowed easily. Thus, the comfortability of taking the preparation is sometimes lowered.

### RELATED ART DOCUMENTS

### PATENT DOCUMENTS

Patent Document 1: JP-2002-534373A (Claims, Paragraph Nos. [0014], [0016])
Patent Document 2: WO 02/087622 (Claims, Examples)

### SUMMARY OF THE INVENTION

### PROBLEMS TO BE SOLVED BY THE INVENTION

It is therefore an object of the present invention to provide an anti-adhesive composition (or a coating agent) which can prevent adhesion of a solid preparation to an inner wall of the oral cavity, a solid preparation comprising a gel-forming layer to which the anti-adhesive composition is applied, and a process for producing the solid preparation.

Another object of the present invention is to provide an anti-adhesive composition (a coating agent) which prevents adhesion of a solid preparation to an inner wall of the oral cavity and can improve the comfortability (or feeling or acceptability) of taking the preparation, a solid preparation comprising a gel-forming layer to which the anti-adhesive composition is applied, and a process for producing the solid preparation.

It is still another object of the present invention to provide a solid preparation which can be swallowed reliably and easily even with a small quantity of water or moisture (such as saliva) while preventing adhesion to an inner wall of the oral cavity effectively, and a process for producing the solid preparation.

### MEANS TO SOLVE THE PROBLEMS

The inventors of the present invention made intensive studies to achieve the above objects and finally found the followings: when an anti-adhesive composition (a coating agent) containing a water-soluble cellulose ether and an anionic polymer is applied and covered (or coated) to a gel-forming layer covering a drug-containing unit, the adhesion of the resulting solid preparation to an inner wall of the oral cavity can be prevented effectively, and the comfortability of taking the solid preparation (or the easy taking of the solid preparation) is improved drastically; particularly, in an anti-adhesive composition for covering a gel-forming layer which comprises a composition containing an anionic polymer, a crosslinking agent, and a base material, when the ratio of the water-soluble cellulose ether relative to the anionic polymer is larger than the ratio of the base material relative to the anionic polymer in the gel-forming layer, a small quantity of water allows the formation of a liquid coat (or membranous layer) on the surface of the gel-forming layer by the anti-adhesive layer with the gel-forming layer forming a strong gel, and thus the adhesion of the solid preparation to the inner wall of the oral cavity can be prevented effectively, and the solid preparation can be swallowed reliably and easily. The present invention was accomplished based on the above findings.

That is, the anti-adhesive composition (coating agent) of the present invention is applied to a gel-forming layer covering a drug-containing unit and prevents adhesion of the gel-forming layer to an inner wall of an oral cavity. The anti-adhesive composition contains a water-soluble cellulose ether and an anionic polymer. The water-soluble cellulose ether may comprise at least one member selected from the group consisting of a methyl cellulose, a hydroxyethyl cellulose, a hydroxyethylmethyl cellulose, a hydroxypropyl cellulose, and a hydroxypropylmethyl cellulose. The water-soluble cellulose ether may have a viscosity of not more than 50 mPa·s for a 5% by mass aqueous solution at 20°C.

The anionic polymer may comprise a homo- or copolymer of (meth) acrylic acid, for example, carboxyvinyl polymer. When the gel-forming layer is formed with a composition containing an anionic polymer, a crosslinking agent, and a base material, the anti-adhesive composition for covering the gel-forming layer practically has a ratio of the water-soluble cellulose ether relative to the anionic polymer being larger than a ratio of the base material relative to the gel-forming agent. The mass ratio of the water-soluble cellulose ether relative to the anionic polymer in the anti-adhesive composition may be about 99.9/0.1 to 85/15 (for example, about 99/1 to 85/15), as the ratio of the water-soluble cellulose ether/the anionic polymer, in terms of a solid content. In order to reduce the viscosity, the anti-adhesive composition may contain a viscosity reducing agent, for example, at least one member selected from the group consisting of a water-soluble metal compound or an electrolyte and a water-soluble organic solvent.

The solid preparation of the present invention comprises a drug-containing unit, a gel-forming layer for covering the drug-containing unit, and an anti-adhesive layer (or a surface layer) for covering the gel-forming layer, wherein the anti-adhesive layer is formed with the anti-adhesive composition. The gel-forming layer may be formed as a crosslinked water-absorbable resin layer (for example, a crosslinked carboxyl-group-containing resin) by crosslinking a gel-forming agent (e.g., a homo- or copolymer of (meth)acrylic acid, such as a carboxyvinyl polymer), and the gel-forming layer swells with absorbing water to form a gel. The gel-forming layer may contain a base material (a film-forming agent). When the gel-forming layer is formed with a composition containing a base material (a film-forming agent), a gel-forming agent (e.g., an anionic polymer), and a crosslinking agent, the ratio of the gel-forming agent (e.g., an anionic polymer) relative to the base material is practically larger than the ratio of the anionic polymer relative to the water-soluble cellulose ether in the anti-adhesive composition. The total thickness of the gel-forming layer and the anti-adhesive layer may be about 5 to 1000 µm, and the ratio of the thickness of the gel-forming layer relative to the thickness of the anti-adhesive layer may be about 15/85 to 50/50 as the ratio of the gel-forming layer/the anti-adhesive layer. Moreover, the solid preparation of the present invention may be a film-covered (or laminate) preparation (for example, a preparation having a shape such as a flat form or a discoid form).

The present invention also includes a process for producing a solid preparation which prevents adhesion of the solid preparation to an inner wall of an oral cavity, the process comprising applying the anti-adhesive composition (or coating agent) to a gel-forming layer covering a drug-containing unit.

In this description, an acrylic monomer and a methacrylic monomer may be referred to as a (meth) acrylic monomer generically. Thus, the (meth)acrylic acid means to include both of acrylic acid and methacrylic acid.

### EFFECTS OF THE INVENTION

According to the present invention, the combination of the gel-forming layer and the specific anti-adhesive layer can prevent adhesion of a solid preparation to an inner wall of the oral cavity effectively. Moreover, the comfortability of taking the solid preparation can drastically be improved compared with conventional preparations. Further, the solid preparation can be swallowed reliably and easily even with a small quantity of water (such as saliva) while preventing adhesion of the preparation to the inner wall of the oral cavity effectively. These advantages are particularly markedly for alarge-sized preparation, a preparation having a flat form and a large contact surface area with the inner wall of the oral cavity, and others.

### BRIEF DESCRIPTION OF DRAWINGS

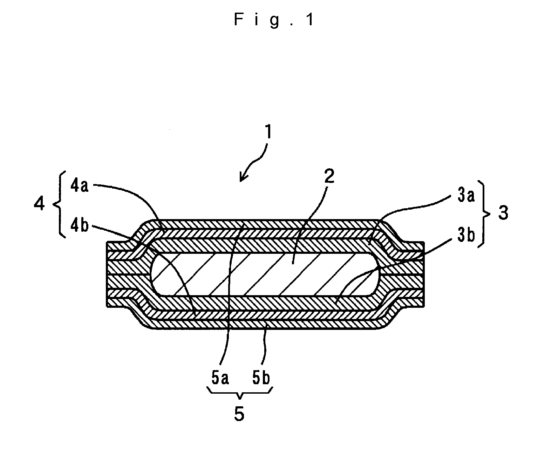

[Fig. 1] Fig. 1 is a schematic cross-sectional view showing a solid preparation in accordance with an embodiment of the present invention.

### DESCRIPTION OF EMBODIMENTS

The solid preparation of the present invention comprises a drug-containing unit, a gel-forming layer for covering the drug-containing unit, and an anti-adhesive agent (or a surface layer) for covering the gel-forming layer and for preventing adhesion of the solid preparation to an inner wall of the oral cavity (or buccal cavity). An adhesive layer may be interposed between the drug-containing unit and the gel-forming layer (in a case where first and second gel-forming layers are extended from the periphery of the drug-containing unit, an adhesive layer maybe interposed between these extended gel-forming layers). Hereinafter, the present invention will be explained in detail with reference to the attached drawings if necessary.

Fig. 1 shows a schematic cross-sectional view of an embodiment of a solid preparation for oral administration (for example, a film-covered (or laminate) preparation) as the solid preparation of the present invention. A solid preparation (for example, a film-covered (or laminate) preparation) 1 comprises a drug-containing unit 2, an adhesive layer 3 for covering the drug-containing unit, a gel-forming layer 4 for covering the adhesive layer, and an anti-adhesive layer 5 for covering the gel-forming layer. In this embodiment, the solid preparation 1 has a laminated form (or structure) . More specifically, the adhesive layer 3 for covering the drug-containing unit 2 comprises a first adhesive layer 3a for covering a first surface of the drug-containing unit 2 and a second adhesive layer 3b for covering a second surface of the drug-containing unit 2. The gel-forming layer 4 comprises a first gel-forming layer 4a for covering the first adhesive layer 3a and a second gel-forming layer 4b for covering the second adhesive layer 3b. The anti-adhesive layer 5 comprises a first anti-adhesive layer 5a for covering the first gel-forming layer 4a and a second anti-adhesive layer 5b for covering the second gel-forming layer 4b. Moreover, the first adhesive layer 3a and the second adhesive layer 3b are joined (or united) with each other at the periphery of the drug-containing unit 2 to form an adhesive layer 3, and the first and second gel-forming layers 4a and 4b are joined (or adhered) to each other through the adhesive layer 3. Incidentally, in this embodiment, the drug-containing unit 2 has a thickness of about 5 µm to 5 mm, the adhesive layer 3 has a thickness of about 1 µm to 1 mm, each of the gel-forming layers 4a and 4b has a thickness of about 1 µm to 1 mm, and each of the anti-adhesive layers 5a and 5b has a thickness of about 1 µm to 50 µm.

According to the solid preparation, even when the preparation has a large flat surface area which contacts with the inner wall of the oral cavity (for example, even when the preparation has a flat form such as a film-like form or a sheet-like form), the preparation can easily be swallowed with a small quantity of water or moisture existing in the oral cavity without adhesion of the preparation to the inner wall of the oral cavity and can have a significantly improved ease (or easiness) of taking the preparation. That is, the conventional solid preparation, particularly a preparation having a flat form, is easily adhered to the inner wall of the oral cavity and has an unpleasant taste, smell, and the like of a drug contained in the preparation. In these respects, the preparation is difficult to swallow and cannot improve the ease of taking the preparation. In contrast, according to the solid preparation of the present invention (for example, a film-covered preparation) 1, the anti-adhesive layer 5 is dissolved by a small quantity of water such as saliva to form a liquid coat on a surface of the solid preparation. Further, the gel-forming layer 4 changes to a gel layer and swells with a small quantity of water (e.g., saliva) in the form enclosing (wrapping) the drug-containing unit 2 to change a shape or surface characteristic of the preparation for significantly improving a slipperiness and an elasticity or viscosity suitable for easy swallowing. The gel layer is slippery to the liquid coat, and thus the preparation can easily be swallowed without adhering the preparation to the palate or other area of the oral cavity. Thus, the solid preparation (for example, a film-covered preparation) 1 can be administered easily and safely even to elderly people and infants (babies and little children), and the medication compliance can extensively be improved. Moreover, the adhesive layer 3 allows adhesion of the gel-forming layer 4 to avoid the exposure of the drug-containing unit in the oral cavity reliably, and the taste (e.g., bitterness) of the drug can be masked reliably.

Hereinafter, each element of the solid preparation will be explained in detail.

### [Drug-containing unit]

The active ingredient (or active component) contained in the drug-containing unit is not particularly limited to a specific one as far as the active ingredient can be orally administered, and, for example, may be either a pharmacologically active ingredient or a physiologically active ingredient, and the pharmacologically active ingredient and the physiologically active ingredient may be used in combination. These ingredients may be solid or semisolid, and as far as the drug-containing unit maintains solid or semisolid forms thereof, a liquid active ingredient may also be used in combination.

There are no particular limitation on the species of the pharmacologically active ingredient, and the pharmacologically active ingredient may for example be a drug which acts to a central nervous system, an autonomic nervous system, a respiratory system, a circulatory system, a digestive system, a metabolic system, or other systems; or may be a drug affecting blood and hemopoiesis, a drug used in the ophthalmologic field or the otological field, an in vivo active substance (autacoid), and others.

As the examples of the pharmacologically active ingredient, there may be mentioned a psychopharmaceutical such as a hypnagogue [for example, a benzodiazepine derivative (e.g., estazolam, triazolam, and nitrazepam), a barbituric acid derivative (e.g., amobarbital and pentobarbital), and zolpidem tartrate], an anxiolytic (for example, etizolam, oxazolam, diazepam, chlordiazepoxide, and hydroxyzine), an antidepressant (for example, maprotiline hydrochloride, amitriptyline hydrochloride, and imipramine hydrochloride), an antivertigo agent (for example, dimenhydrinate, isoprenaline hydrochloride, difenidol hydrochloride, and betahistine mesilate), or an antipsychotic agent (for example, perphenazine, chlorpromazine, sulpiride, and haloperidol); an antiparkinson agent (for example, levodopa, pergolide mesilate, trihexyphenidyl, amantadine hydrochloride, and droxidopa); an analgesic and an antiphlogistic (or an antiinflammatory agent) (for example, pentazocine, sulpyrine, acetaminophen, salicylic acid,flufenamic acid, mefenamic acid, tolfenamic acid, diclofenac sodium, indometacin, ibuprofen, ketoprofen, piroxicam, acetaminophen, aspirin, isopropylantipyrine, streptokinase, streptodornase, serrapeptase, and pronase); an antirheumatic (for example, penicillamine); a hyperlithuria-treating agent (for example, benzbromarone and allopurinol); an agent for treating gout (for example, allopurinol, probenecid, and colchicine); a central nerve system metabolism activator (or a cerebral circulation and metabolism improver) (for example, adenosine triphosphate (ATP), γ-aminobutyric acid, meclofenoxaet hydrochloride, tiapride hydrochloride, and ifenprodil tartrate);a antihistamine (for example, diphenhydramine hydrochloride, chlorpheniramine maleate, and clemastine fumarate); an agent for treating allergy (or an antiallergic agent) (for example, sodium cromoglicate, tranilast, amlexanox, ketotifen fumarate, azelastine hydrochloride, oxatomide, ebastine, pranlukast, fexofenadine hydrochloride, and loratadine); a cardiant (for example, digitoxin, digoxin, dopamine hydrochloride, epinephrine, aminophylline, and caffeine); an antianginal agent (for example, amyl nitrite, isosorbide dinitrate, nicorandil, dipyridamole, trapidil, trimetazidine hydrochloride, dilazep hydrochloride, and nitroglycerin); a β-blocking agent (for example, propranolol hydrochloride, alprenolol hydrochloride, atenolol, metoprolol tartrate, and labetalol hydrochloride); a Ca antagonist (for example, nifedipine, nicardipine, manidipine hydrochloride, verapamil hydrochloride, diltiazem hydrochloride, amlodipine besilate, and verapamil hydrochloride); an antiarrhythmic agent (for example, quinidine sulfate, ajmaline, disopyramide, procainamide hydrochloride, lidocaine hydrochloride, mexiletine hydrochloride, and sotalol hydrochloride); a diuretic (for example, trichlormethiazide, hydrochlorothiazide, furosemide, spironolactone, triamterene, and acetazolamide); an antihypertensive (for example, methyldopa, clonidine hydrochloride, prazosin hydrochloride, hydralazine hydrochloride, enalapril maleate, captopril, reserpine, and losartan); a hyperlipidemia-treating agent such as an HMG-CoA reductase inhibitor (for example, pravastatin, simvastatin, fluvastatin, and atorvastatin), a fibrate-series agent (for example, clofibrate, clinofibrate, simfibrate, bezafibrate, and fenofibrate), nicotinic acid and a derivative thereof (for example, nicomolandniceritrol),probucol,ordextransulfatesodium sulfur; a hypotension-treating agent (for example, metaraminol bitartrate and etilefrine hydrochloride); a bronchodilator or an antasthmatic (for example, epinephrine, ephedrine hydrochloride, salbutamol sulfate, terbutaline sulfate, formoterol fumarate, procaterol hydrochloride, fluticasonepropionate, theophylline, and aminophylline); an antitussive (for example, codeine phosphate, dimemorfan phosphate, dextromethorphan hydrobromide, and chloperastine); an expectorant (for example, bromhexine hydrochloride, carbocisteine, and ambroxol hydrochloride);an antiulcer agent(forexample,cimetidine, ranitidine hydrochloride, famotidine, omeprazole, lansoprazole, secretin, sucralfate, azulene, aldioxa, teprenone, rebamipide, and cetraxate hydrochloride);a stomachic and digestant (or an antiemetic) (for example, carnitine chloride, domperidone, metoclopramide, gasmotin, trimebutine maleate, and a digestive enzyme) ; a cathartic (or a purgative) (for example, bisacodyl and sennoside); an antidiarrhetic and antiflatuent agent (for example, loperamide hydrochloride, berberine chloride, tilactase, mepenzolate bromide, sulfasalazine, and lactobacillus bifidus) ; an agent for treating hepatic disease (for example, glutathione, sodium glucuronate, glucuronolactone, and a liver extract preparation) ; a diabetic agent (for example, tolbutamide, chlorpropamide, glibenclamide, metformin hydrochloride, pioglitazone hydrochloride, voglibose, and glimepiride); a styptic (for example, carbazochrome sodium sulfonate and tranexamic acid) ; an agent for treating anemia (for example, ferrous sulfate and sodium ferrous citrate); an antithrombotic agent (for example, ticlopidine hydrochloride, cilostazol, and warfarin potassium); an antibiotic (for example, erythromycin stearate, cefaclor, fosfomycin, minocycline hydrochloride, rokitamycin, azithromycin, and lincomycin); a chemotherapeutic agent (for example, ofloxacin, norfloxacin, isoniazid, rifampicin, and ethambutol hydrochloride); an anticancer agent (for example, cyclophosphamide, methotrexate, fluorouracil, tegafur, etoposide, and bicalutamide); an immunosuppressant (for example, azathioprine and tacrolimushydrate); a hormone and a nendocrine-therapeutic agent (for example, kallidinogenase (as a peripheral vasodilating agent), a corpus luteum hormone, a salivary gland hormone, thiamazole, prednisolone, betamethasone, and levothyroxine); a vitamin compound (such as vitamin A, D, B₁, B₂, B₆, B₁₂, C, or E) (for example, retinol palmitate, alfacalcidol, thiamin hydrochloride, fursultiamine, octotiamine, bisbentiamine, benfotiamine, riboflavin, pyridoxine hydrochloride, nicotinic acid, nicotinamide, cyanocobalamin, cobamamide, mecobalamin, folic acid, pantothenic acid, ascorbic acid, tocopherol succinate, tocopherol nicotinate, and biotin) ; and a crude drug. These ingredients may be used alone or in combination according to the purposes of prevention, treatment, and others.

Examples of the physiologically active ingredient may include an organic acid or a salt thereof [for example, α-lipoic acid, L-ascorbic acid, citric acid, malic acid, tartaric acid, oxalic acid, and fumaric acid, or an alkali metal salt thereof (e. g., a sodium salt and a calcium salt)], an amino acid or a salt thereof [for example, glycine, L-lysine, L-valine, L-alanine, L-arginine, L-cystine, L-methionine, L-glutamic acid, and L-aspartic acid, or an alkali metal salt thereof (e.g., a sodium salt)], a peptide or a salt thereof [for example, a peptide (such as L-lysineglutamate or a collagen and a collagen peptide thereof), coenzyme Q₁₀, and L-carnitine or a salt thereof (such as fumarate or tartrate)], a glycosaminoglycan compound (for example, chondroitin, sodium chondroitin sulfate, and hyaluronic acid), a polyphenol compound (for example, tea catechin and soybean isoflavone), a ceramide compound (for example, wheat, rice, and soybean ceramide), a plant powder or extract (for example, turmeric powder, garcinia cambogia powder, gymnema sylvestre powder, senna stem powder, and aloe powder, and an extract thereof), a polysaccharide (for example, a glucan derived from fungus, such as a fungus belonging to Polyporacease), a glucosamine compound (for example, chitin and chitosan), a mineral compound (for example, calcium, iron, and table salt), and a yeast (for example, beer yeast). These physiologically active ingredients may be used alone or in combination.

According to the present invention, since the drug-containing unit can be enclosed in the gel-forming layer and the anti-adhesive layer, a physical strength can be imparted to the solid preparation even when the solid preparation contains a relatively large amount of an active ingredient, or a bulky active ingredient, which easily lowers the physical strength of the solid preparation. Thus, the present invention can be applied to both a slight or low dose (e.g., not more than 1 mg) of an active ingredient and a large or high dose (e.g., not less than 300 mg) of an active ingredient as the active ingredient. The unit dosage amount of the active ingredient may for example be about 0.01 to 1500mg (e.g., about 0.01 to 800mg), preferably about 0.1 to 1200 mg (e.g., about 0.1 to 500 mg), and more preferably about 1 to 1000 mg (e.g., about 1 to 300 mg) and is usually about 1 to 500 mg (e.g., about 2 to 250 mg). The active ingredient content can be selected according to the species of the active ingredient or others, and is usually, in the drug-containing unit, about 0.001 to 100% by mass, preferably about 0.01 to 70% by mass (e.g., about 0.01 to 50% by mass), and more preferably about 0.1 to 35% by mass.

The solid preparation of the present invention provides a comfortable feeling (or great ease) to take and can effectively be administered orally with a small quantity of water or substantially without water. Thus, for example, the solid preparation can suitably be used for an active ingredient having a large unit dosage amount, a bulky active ingredient, an unpalatable (such as bitter or acerbic) active ingredient, a highly water-soluble active ingredient. Among these ingredients, usually, the pharmacologically active ingredient is widely used.

The drug-containing unit may comprise the active ingredient, and usually contains an additive (a base material or a carrier). The additive is not particularly limited to a specific one, and depending on the shape of the preparation, a conventional carrier, for example, at least one carrier selected from the group consisting of an excipient, a binder, a disintegrant, and a lubricant may be selected.

As the excipient, there may be mentioned a saccharide such as lactose, white sugar or refined sugar, maltose, glucose, sucrose, or fructose (or fruit sugar); a sugar alcohol such as mannitol, sorbitol, or xylitol; a starch such as a corn starch or a potato starch; a polysaccharide such as a crystalline cellulose (including a microcrystalline cellulose), cyclodextrin, or dextran; silicon dioxide or a silicate such as a light silicic anhydride, a synthetic aluminum silicate, magnesium silicate, magnesium aluminometasilicate, or atalc; an oxide such as titanium oxide; a carbonate such as calcium carbonate or magnesium carbonate; a phosphate such as calcium monohydrogenphosphate; and others. The binder may include a water-soluble starch or starch derivative such as a pregelatinized starch, a partially pregelatinized starch, an oxidized starch, a sodium carboxymethyl starch, a hydroxypropyl starch, or dextrin; a polysaccharide such as agar, gum acacia (or gumarabic), dextrin, sodiumalginate, a tragacanth gum, a pullulan, a xanthan gum, a hyaluronic acid, a pectin, a sodium chondroitin sulfate, or a gelatin; a synthetic polymer such as a polyvinylpyrrolidone (e.g., a povidone), a vinyl acetate-vinylpyrrolidone copolymer, a poly(vinylalcohol), a carboxyvinyl polymer, a polyacrylic acid-series polymer, a polylactic acid, a poly(ethylene glycol), or a poly(vinyl acetate); a cellulose ether such as a methyl cellulose (MC), an ethyl cellulose (EC), a carboxymethyl cellulose (CMC), a carboxymethylethyl cellulose (CMEC), a hydroxypropyl cellulose (HPC), or a hydroxypropylmethyl cellulose (HPMC), and a cellulose ester such as a cellulose acetate; and others. The disintegrant may include calcium carbonate, a carboxymethyl cellulose or a salt thereof (e.g., a carmellose, a carmellose sodium, a carmellose calcium, and a croscarmellose sodium), a polyvinylpyrrolidone (e.g., a povidone and a crosslinked polyvinylpyrrolidone (crospovidone)), a low-substituted hydroxypropyl cellulose, magnesium aluminometasilicate, and others. The lubricant may include a talc, magnesium stearate, a poly(ethylene glycol) 6000, and others. These carriers may be used alone or in combination.

The drug-containing unit may contain a polyglucosamine compound (such as a chitin or a chitosan), a protein (such as a casein or a soybean protein), an enteric base material (e.g., a cellulose derivative such as a cellulose phthalate, a cellulose acetate phthalate, a hydroxypropyl cellulose phthalate, a hydroxypropylmethyl cellulose phthalate (HPMCF), or a hydroxypropylmethyl acetate succinate, a methacrylic acid-ethyl acrylate copolymer (methacrylic acid copolymer LD), a methacrylic acid-n-butyl acrylate copolymer, and a methacrylic acid-methyl methacrylate copolymer (methacrylic acid copolymers L and S)), a gastric-soluble base material (a dimethylaminoethyl methacrylate-methacrylic acid copolymer, a dimethylaminoethyl methacrylate-methyl methacrylate copolymer, a dimethylaminoethyl methacrylate-chlorotrimethylammoniumethyl methacrylate copolymer, a dimethylaminoethyl methacrylate-chlorotrimethylammoniummethyl methacrylate copolymer, a dimethylaminoethyl methacrylate-chlorotrimethylammoniumethyl acrylate copolymer, a polyvinylacetal diethylaminoacetate), and others. Moreover, the enteric base material and/or gastric-soluble base material may be used as the binder.

Further, the drug-containing unit may contain a lipid. The lipid may include a wax (e.g., a bees wax, a carnauba wax, a cacao butter, a lanolin, a paraffin, and a petrolatum), a higher (or long chain) fatty acid ester [e.g., an alkyl ester of a saturated or unsaturated fatty acid, and an ester of a fatty acid with a polyhydric alcohol (such as a poly(C₂₋₄alkylene glycol), glycerin, or a polyglycerin) (e.g., a glyceride)], a hardened (or hydrogenated) oil, a higher alcohol (e.g., a saturated aliphatic alcohol such as stearyl alcohol and an unsaturated aliphatic alcohol such as oleyl alcohol), a higher fatty acid (e.g., linoleic acid, linolenic acid, oleic acid, and stearic acid), a metallic soap (e.g., a metal salt of a fatty acid, such as a sodium salt of palm oil fatty acid or calcium stearate), and others.

Furthermore, for the drug-containing unit, a known additive can be used. Such an additive may include, for example, a disintegrant aid (or adjuvant), an antioxidation agent or an antioxidant, a variety of surfactants such as a nonionic surfactant), a dispersing agent, an antiseptic agent or a preservative (e.g., a paraben such as methyl paraben or butyl paraben), a fungicide or antibacterial agent (e.g., abenzoic acid compound such as sodiumbenzoate), an antistatic agent, a corrigent or a masking agent (e.g., sweetening agent), a coloring agent (e.g., a dye and a pigment such as titanium oxide or colcothar), a deodorant or a flavoring agent (or perfume) (e.g., an aromatic substance), and an algefacient. These additives may be used alone or in combination.

The ratio of the additive may for example be about 0.001 to 100 parts by mass (e.g., about 0.01 to 50 parts by mass, preferably about 0.1 to 30 parts by mass, and more preferably about 0.5 to 20 parts by mass) relative to 1 part by mass of the active ingredient.

The drug-containing unit containing the active ingredient and the additive (base material or carrier) may be shaped or formed into various shapes or dosage forms of solid preparations, for example, powdered preparations, powders, granulated preparations (e.g., granules and microfine granules), spherical or spheroidal preparations, tablets, capsules (including hard capsules, soft capsules, and microcapsules), and layered or film-covered preparations (or sheet-shaped preparations). The shape (or form) of the drug-containing unit may for example be a spherical shape, an ellipsoidal shape, a polyhedral or prismatic shape, a layered shape, an amorphous shape, and an aggregate of particles.

According to the present invention, even when the solid preparation has a large contact surface area with the inner wall of the oral cavity due to the shape of the preparation, the solid preparation can easily be swallowed without water or with a small quantity of water, and the comfortability of taking the preparation can be improved. Moreover, the preparation can easily be swallowed even in spite of a high drug content and a large dosage size. Thus, the drug-containing unit may be formed as a preparation that is conventionally difficult for elderly people and infants (babies and little children) to swallow [for example, a preparation having a flat region or plateau, a preparation having a flat shape, and a large-sized tablet (e.g., a tablet having a diameter of about 5 to 15 mm, preferably about 6 to 14 mm, and more preferably about 7 to 13 mm)]. Among these shapes, the drug-containing unit may have a layered or film-like shape (e.g., a polygon such as a quadrilateral, a circle, and an ellipse). The layered drug-containing unit may for example have a thickness of about 5 µm to 5 mm, preferably about 10 µm to 3 mm, and more preferably about 100 to 1000 µm (e.g., about 100 to 500 µm).

### [Adhesive layer (or intermediate layer)]

An adhesive layer (or intermediate layer) is not necessarily required between the drug-containing unit and the gel-forming layer. However, when the first and second gel-forming layers are joined (or adhered) together through the adhesive layer (or intermediate layer) at the periphery of the drug-containing unit, the adhesive layer intimately joins (or adheres) these gel-forming layers to each other, effectively prevents leakage of the active ingredient from the drug-containing unit, and allows smooth administration of the preparation.

The base material (adhesive) of the adhesive layer (or intermediate layer) may be either a water-soluble adhesive or a water-insoluble adhesive. As the water-soluble adhesive, there may be mentioned a (meth)acrylic acid-series polymer [for example, a polyacrylic acid or a salt thereof (such as a carboxyvinyl polymer or a poly(sodium acrylate)); and an acrylic acid copolymer or a salt thereof], a vinylpyrrolidone-series polymer [a povidone, and a copolymer of vinylpyrrolidone such as a vinyl acetate-vinylpyrrolidone copolymer], a polysaccharide [for example, a polysaccharide derived from a plant (e.g., a cellulose derivative such as a CMC, a CMC sodium salt, an MC, an HPC, or an HPMC, a karaya gum, a pectin, a tragacanth gum, alginic acid, and a gum acacia (or gum arable)), and a polysaccharide derived from a fungus (e.g., an acidic polysaccharide such as a pullulan, a karaya gum, a pectin, a xanthan gum, a gum acacia (or gum arabic), a tragacanth gum, alginic acid or a sodium salt thereof, a hyaluronic acid, or a chondroitin sulfate or a sodium salt thereof)], and others. Examples of the water-insoluble adhesive (for example, an adhesive soluble in an organic solvent such as ethanol or acetone) may include a vinyl acetate-series polymer (e.g., a poly((vinyl acetate) and an ethylene-vinyl acetate copolymer), a (meth) acrylic acid-series polymer [e.g., a methacrylic acid-ethyl acrylate copolymer (methacrylic acid copolymer LD), a methacrylic acid-n-butyl acrylate copolymer, and a methacrylic acid-methyl methacrylate copolymer (methacrylic acid copolymers L and S)], and others. The water-soluble (meth)acrylic acid-series polymer may include the same polymer as the after-mentioned gel-forming agent or anionic polymer for the anti-adhesive composition. These adhesives may be used alone or in combination.

The adhesive may have heat (or thermal) adhesiveness (heat sealing property). Such an adhesive having heat adhesiveness may include a (meth)acrylic acid-series polymer, a vinylpyrrolidone-series polymer, a vinyl acetate-series polymer, and others.

As the adhesive, a water-soluble polymer is practically used, and there may be mentioned a (meth) acrylic acid-series polymer (such as a carboxyvinyl polymer) and a vinylpyrrolidone-series polymer (such as a povidone) as the water-soluble polymer. Moreover, when an adhesive having both water solubility and heat adhesiveness is used, the drug-containing unit can be sealed in a simple operation by interposing the drug-containing unit between a pair of film-like adhesive layers and heat-adhering (heat-bonding) the adhesive layers each other at the periphery of the drug-containing unit.

The adhesive layer may contain a plasticizer. Examples of the plasticizer may include a water-soluble plasticizer [e.g., ethylene glycol, propylene glycol, glycerin, sorbitol, sucrose, a polyoxyethylene polyoxypropylene glycol (such as pluronic or poloxamer), a polyoxyethylene sorbitan fatty acid ester (such as polysorbate 80), and a poly(ethylene glycol) (such as macrogol 400, 600, 1500, 4000, or 6000)], a water-insoluble plasticizer (e.g., triacetin, triethyl citrate, diethyl phthalate, dioctyl adipate, and a fatty acid such as lauric acid), and others. These plasticizers may be used alone or in combination. The preferred plasticizer includes a water-soluble plasticizer, such as glycerin.

The amount of the plasticizer may be selected according to the species of the base material (adhesive) of the adhesive layer, and may be about 1 to 100 parts by mass, preferably about 5 to 75 parts by mass (e.g., about 10 to 50 parts by mass), and more preferably about 15 to 50 parts by mass (e.g., about 20 to 40 parts by mass) relative to 100 parts by mass of the base material.

The adhesive layer may cover (or coat) the whole or at least part of the surface of the drug-containing unit to adhere (or bond) the drug-containing unit to the gel-forming layer. The adhesive layer may usually cover (or coat) the whole or part of the surface of the drug-containing unit (for example, at least upper and under surfaces of a layered drug-containing unit).

The thickness of the adhesive layer may be selected from a wide range of, for example, about 1 µm to 1 mm (e.g., about 5 to 500 µm) as far as the drug-containing unit is not exposed. The thickness of the adhesive layer may be about 10 to 500 µm (e.g., about 15 to 300 µm), preferably about 20 to 200 µm (e.g., about 30 to 175 µm), and more preferably about 50 to 150 µm.

### [Gel-forming layer]

The gel-forming layer encloses (or wraps) the drug-containing unit and gelates by a small quantity of water such as saliva, so that the gel-forming layer changes a shape or surface characteristic of the preparation to impart a significantly improved slipperiness and an elasticity or viscosity suitable for easy swallowing to the preparation. Thus the comfortability (or feeling) of taking the preparation is improved (for example, the gel-forming layer facilitates the swallowing of the preparation).
There are no particular limitations on the gel-forming agent of the gel-forming layer as far as the agent is pharmaceutically acceptable, and may be a synthetic polymer, a cellulose derivative, a starch derivative, a protein (e.g., a collagen and a casein), a natural polysaccharide, and others. As the gel-forming agent, there may be mentioned a hydroxyl group-containing polymer (or macromolecule) (e.g., a synthetic polymer such as a poly(vinyl alcohol), a cellulose derivative such as an MC, an HPC, or an HPMC, a starch derivative such as a hydroxypropyl starch or a dextrin, and a natural polysaccharide such as an agar, a galactomannan, a glucomannan, a guar gum, a locust bean gum, a gum acacia (or gum arabic), an arabinogalactan, a tamarind gum, a psyllium seed gum, or a dextran), a carboxyl group-containing polymer (or macromolecule) [a synthetic polymer such as a carboxyl group-containing polymer obtainable from at least one polymerizable monomer selected from the group consisting of (meth) acrylic acid and itaconic acid as a polymerizable component, or a carboxyvinyl polymer; a cellulose derivative such as a CMC, a carboxymethylethyl cellulose, or a carboxymethylhydroxyethyl cellulose; a starch derivative such as a carboxymethyl starch; and a natural polysaccharide such as alginic acid, a heparin, a hyaluronic acid, a pectin, a tragacanth gum, a xanthan gum, or a gellan gum], a sulfonic acid group-containing polymer [e.g., a synthetic polymer such as a poly(styrene sulfonic acid), a poly(ethylene sulfonic acid), a poly(vinyl sulfate), a cellulose derivative such as a cellulose sulfate, and a natural polysaccharide such as a hyaluronic acid, a carrageenan, or a chondroitin sulfate], a phosphoric acid group-containing polymer (e.g., a cellulose derivative such as a cellulose phosphate), or a salt thereof, and others. Incidentally, the cellulose derivative, the starch derivative and the natural polysaccharide, each having a carboxyl group or a sulfonic acid group, also have a hydroxyl group. Therefore, these components can also be classified into the hydroxyl group-containing polymer. These gel-forming agents may be used alone or in combination.

The anionic polymer (e.g., the carboxyl group-containing polymer, the sulfonic acid group-containing polymer, and the phosphoric acid group-containing polymer) may form, for example, a salt with an inorganic base [e. g., an alkali metal (such as sodium or potassium), and ammonia], an organic base [e.g., monoethanolamine, diethanolamine, triethanolamine, and dimethylaminoethanol].

Among these gel-forming agents, in order to absorb water or moisture rapidly, it is preferred to use an anionic polymer (particularly, a water-soluble anionic polymer), for example, a carboxy group-containing polymer and a sulfonic acid group-containing polymer, particularly an anionic polymer comprising a (meth)acrylic acid unit as an essential polymerizable component (a homo- or copolymer of (meth)acrylic acid, or a (meth)acrylic acid-series polymer). As a monomer copolymerized with (meth)acrylic acid (copolymerizable monomer), there may be mentioned an alkyl (meth)acrylate [for example, a C₁₋₆alkyl (meth)acrylate such as methyl (meth)acrylate, ethyl (meth)acrylate, or butyl (meth)acrylate, particularly a C₁₋₄alkyl (meth)acrylate], a hydroxyalkyl (meth)acrylate [for example, a hydroxyC₂₋₄alkyl (meth)acrylate such as hydroxyethyl (meth)acrylate or hydroxypropyl (meth)acrylate, particularly a hydroxyC₂₋₃alkyl (meth)acrylate], vinyl acetate, vinylpyrrolidone, and others. These copolymerizable monomers may be used alone or in combination.

The mass ratio of the (meth) acrylic acid relative to the copolymerizable monomer may for example be about 100/0 to 50/50, preferably about 100/0 to 60/40 (e.g., about 99.9/0.1 to 65/35), andmore preferably about 100/0 to 70/30 (e.g., about 99/1 to 80/20), as the (meth) acrylic acid/the copolymerizable monomer.

The (meth) acrylic acid-series polymer may include a poly((meth)acrylic acid), a (meth)acrylic acid-methyl (meth)acrylate copolymer, a (meth)acrylic acid-ethyl (meth)acrylate copolymer, a (meth)acrylic acid-butyl (meth) acrylate copolymer, and others. These(meth)acrylic acid-series polymers may be used alone or in combination.

Representative examples of the (meth)acrylic acid-series polymer includes a carboxyvinyl polymer (trade name: CARBOPOL), a poly(sodium acrylate), a partially neutralized product of a polyacrylic acid, a methacrylic acid-n-butyl acrylate copolymer, and a methacrylic acid copolymer LD (trade name: EUDRAGIT L-30D55). Among these (meth) acrylic acid-series polymers, a polyacrylic acid or an acrylic acid copolymer in each of which acrylic acid as a main monomer is polymerized (that is, an acrylic acid-series polymer), particularly a carboxyvinyl polymer, is preferred. As the carboxyvinyl polymer, there may be mentioned CARBOPOL 981, CARBOPOL 980, CARBOPOL 974P, CARBOPOL 971P, CARBOPOL 941, CARBOPOL 940, CARBOPOL 934P, CARBOPOL 71G (manufactured by Noveon, US), HIVISWAKO 103, HIVISWAKO 104 (manuf acturedbywako Pure Chemical Industries, Ltd.), JUNLON (Nihon Junyaku Co., Ltd.), AQUPEC (Sumitomo Seika Chemicals Company Limited), and others.

The gel-forming agent (e.g., a carboxyvinyl polymer) may have a viscosity of about 1500 to 50000 mPa·s, preferably about 2500 to 20000 mPa·s, more preferably about 5000 to 15000 mPa·s, and particularly about 7500 to 12500 mPa·s (e.g., 8000 to 12000 mPa·s) for a 0.2% by mass aqueous solution at 20°C.

The gel-forming agent content of the gel-forming layer may be selected from a range in which the gel-forming agent can absorb water rapidly to form a gel and inhibit the dissolution of the gel-forming agent and may for example be about 5 to 90% by mass (e.g., about 10 to 80% by mass) in terms of a non-volatile matter. The gel-forming agent content of the gel-forming layer may be about 10 to 70% by mass (e.g., about 12 to 50% by mass) and preferably about 15 to 35% by mass (e.g., about 15 to 25% by mass) in terms of a non-volatile matter relative to the whole gel-forming layer.

The gel-forming layer may contain a pharmaceutically acceptable base material or a film-forming agent. The base material (film-forming agent) inhibits cracks of the gel-forming layer, stabilizes the shape of the gel-forming layer, and prevents the separation of the gel from the drug-containing unit.

Examples of the base material (film-forming agent) may include a vinyl-series polymer [for example, a (meth)acrylicpolymer, avinylalcohol-seriespolymer (such as a poly(vinyl alcohol)), a vinylpyrrolidone-series polymer (such as a povidone or a vinyl acetate-vinylpyrrolidone copolymer), a poly(vinyl acetate), and a poly(vinyl acetate phthalate)], a poly(ethylene glycol), and a polysaccharide derived from a plant [for example, a cellulose ether (e.g., an MC, a hydroxymethyl cellulose (HMC), an HEC, an HPC, and an HPMC), a xanthan gum, and a carrageenan]. These components may be used alone or in combination.

Among these film-forming agents, a water-soluble base material [for example, a poly(vinyl alcohol), a vinylpyrrolidone-series polymer, and a cellulose ether] is preferred. Use of the water-soluble base material facilitates the permeation (or infiltration) of water in the gel-forming layer, and the gel-forming layer can rapidly swell in the oral cavity to form a gel. In particular, use of the vinyl alcohol-series polymer (e.g., a poly(vinyl alcohol)) is useful for shielding and masking unpleasant taste and smell of the active ingredient contained in the drug-containing unit.

The base material content of the whole gel-forming layer may be selected from the range of about 20 to 85% by mass (e.g., about 30 to 80% by mass) and may usually be about 50 to 85% by mass and preferably about 60 to 80% by mass (e.g., about 65 to 75% by mass).

The mass ratio of the base material (film-forming agent) relative to the gel-forming agent (e.g., an anionic polymer) may be selected from the range of about 99/1 to 10/90 (e.g., about 90/10 to 15/85, particularly about 85/15 to 20/80) in terms of a solid content, and may usually be about 85/15 to 50/50 (e.g., about 82.5/17.5 to 65/35) and preferably about 80/20 to 70/30, as the base material/the gel-forming agent. The ratio of the base material relative to 100 parts by mass of the gel-forming agent may for example be about 50 to 700 parts by mass (e.g., about 100 to 500 parts by mass), preferably about 200 to 400 parts by mass, and more preferably about 250 to 350 parts by mass.

The gel-forming layer can for example be formed as a crosslinked gel-forming layer obtainable from a composition containing the gel-forming agent and a crosslinking agent. The crosslinked gel layer can form a gel having a high strength even in swelling due to water absorption, and having an elasticity and a high slipperiness in the oral cavity. Such a gel facilitates swallowing of the solid preparation and prevents dissolution in the oral cavity.

The crosslinking agent may be selected according to the species of the gel-forming agent, and as the crosslinking agent for the anionic polymer, for example, a polyvalent metal compound can be used. The polyvalent metal compound is not particularly limited to a specific one as far as the compound is a pharmaceutically acceptable metal compound. Such a metal compound may include, for example, a polyvalent metal salt, a polyvalent metal oxide, a polyvalent metal hydroxide, and a polyvalent metal carbonate. Examples of the polyvalent metal may include an alkaline earth metal [for example, magnesium and calcium], and metals of the groups 3 to 13 of the Periodic Table of Elements [for example, a metal of the group 8 of the Periodic Table of Elements (e.g., iron), a metal of the group 12 of the Periodic Table of Elements (e.g., zinc), and a metal of the group 13 of the Periodic Table of Elements (e.g., aluminum)].

As these polyvalent metal compounds, for example, there may be mentioned calcium oxide, calcium chloride, magnesium oxide, magnesium chloride, zinc oxide, zinc sulfate, ferric sulfate, iron citrate, aluminum chloride, aluminum hydroxide, aluminum sulfate, aluminum silicate, aluminum phosphate, and an alum compound (for example, aluminum potassium sulfate (potassium alum), ammonium ion (III) sulfate dodecahydrate (ammonium iron alum), and aluminum ammonium sulfate (ammonium alum)). These polyvalent metal compounds may be used alone or in combination. Incidentally, use of a trivalent metal compound increases the degree of crosslinking of the gel-forming agent to improve the physical strength of the gel-forming layer and to prevent the dissolution of the gel-forming agent certainly (or surely).

Regarding the ratio (mass ratio) of the gel-forming agent relative to the crosslinking agent, the ratio of the crosslinking agent relative to 100 parts by mass of the gel-forming agent is, for example, about 0.1 to 10 parts by mass (e.g., about 0.5 to 7.5 parts by mass), preferably about 1 to 5 parts by mass, and more preferably about 1.5 to 3.5 parts by mass (e.g., about 2 to 3 parts by mass). The crosslinking of the gel-forming agent with the crosslinking agent can retain the form (or shape) of the gel-forming layer while preventing the dissolution of the gel-forming layer. Moreover, a viscosity of a liquid coating composition as a material of the gel-forming layer can be lowered by regulating the ratio of the gel-forming agent and the crosslinking agent to form the gel-forming layer further efficiently.

Further, the ratio of the crosslinking agent relative to 100 parts by mass of the total amount of the base material and the gel-forming agent may for example be about 0.1 to 2.5 parts by mass, preferably about 0.2 to 1.5 parts by mass (e.g., about 0.25 to 1.2 parts by mass) , and more preferably about 0.3 to 1 parts by mass (e.g., about 0.5 to 0.8 parts by mass).

In order to increase the water-absorption speed and gelation speed, the gel-forming layer may contain a water absorption promoter. As the water absorption promoter, there may be used a highly water-soluble component. Examples of the water absorption promoter may include a monosaccharide or a disaccharide (for example, glucose, xylose, mannose, fructose, galactose, sucrose, fruit sugar (or levulose), and white sugar or refined sugar), a polyhydric alcohol [for example, an alkanediol (e.g., propylene glycol), a poly(ethylene glycol) (e.g., a poly(ethylene glycol) such as macrogol 300, macrogol 400, macrogol 600, macrogol 1500, macrogol 4000, or macrogol 20000; and a polyoxyethylene polyoxypropylene glycol), and a polyol having three or more hydroxyl groups (a tri- to polyvalent polyol) (e.g.,glycerin),a sugar alcohol (e.g., erythritol, sorbitol, xylitol, mannitol, inositol, maltitol, and lactitol)], and an ethylene oxide adduct (e.g., polyoxyl 40 stearate, polyoxyl 45 stearate, polyoxyl 55 stearate, and polyoxyethylene hydrogenated castor oil). These water absorption promoters may be used alone or in combination.

Among these water absorption promoters, the polyhydric alcohol, particularly glycerin, is preferred, since the polyhydric alcohol has an excellent ability to accelerate water absorption and imparts flexibility to the gel to further ease swallowing of the solid preparation. Moreover, the monosaccharide or the disaccharide, the sugar alcohol or the glycerin can also mask the bitterness, acerbity and other unpleasant tastes of the drug.

The water absorption promoter may have a viscosity of about 0.3 to 5.0 mPa·s, preferably about 0.5 to 3.5 mPa·s, and more preferably about 0.6 to 2.5 mPa·s (e.g., about 0.6 to 2 mPa·s) for a 5% by mass aqueous solution at 37°C, and may have a viscosity of about 0.6 to 1.8 mPa·s for a 5% by mass aqueous solution at 37°C. The lower the viscosity of the aqueous solution of the water absorption promoter is, the higher the water-absorption speed of the gel-forming layer is.

From the point of view of form (or shape) retention and water absorption (percentage of water absorption) of the gel, the mass ratio of the water absorption promoter relative to 100 parts by mass of the gel-forming agent may be about 1 to 100 parts by mass, preferably about 5 to 75 parts by mass, and more preferably about 10 to 50 parts by mass (e.g., about 25 to 50 parts by mass). Incidentally, when a plurality of water absorption promoters containing glycerin is used, the glycerin content of the whole water absorption promoter may be about 35 to 95% by mass and preferably about 40 to 90% by mass.

The gel-forming layer may contain various optional components, for example, a plasticizer, a masking agent, an antiseptic agent, and a coloring agent, as with the after-mentioned anti-adhesive layer.

It is sufficient that the gel-forming layer covers at least part of the surface of the drug-containing unit (or the surface of the adhesive layer when the solid preparation contains the adhesive layer), particularly, the whole or the most of the surface thereof (for example, about 50 to 100% and preferably about 80 to 100%). The gel-forming layer may cover the surface area of the drug-containing unit or that of the adhesive layer uniformly or nonuniformly (scatteringly in a polygonal pattern such as quadrilateral pattern, a circular pattern, or a grid pattern). The gel-forming layer usually covers the whole of the drug-containing unit or that the adhesive layer (in the above-mentioned embodiment, at least upper and under surfaces).

The thickness of the gel-forming layer may be selected from the range of, for example, about 1 to 1000 µm (e.g., about 3 to 700 µm) and may be about 5 to 500 µm, and preferably about 7 to 250 µm (e.g., about 10 to 100 µm). Even a layer having a thickness of about 5 to 50 µm (e.g., about 10 to 30 µm) performs a sufficient function as the gel-forming layer. Incidentally, when the gel-forming layer is prepared, a plurality of thin gel-forming layers [gel-forming layers, each having a thickness of not more than 10 µm (e.g., about 1 to 10 µm, preferably about 2 to 9 µm, and more preferably about 3 to8 µm)] may be laminated (or layered) to form a gel-forming layer having a predetermined thickness, thereby accelerating the gelation speed, according to a method described in Japanese Patent Application Laid-Open No. 2008-37794.

### [Anti-adhesive layer or anti-adhesive composition]

The anti-adhesive layer may be water-insoluble, and is preferably water-soluble in order to rapidly absorb water in the oral cavity. The anti-adhesive composition or the anti-adhesive layer (surface layer) for covering the gel-forming layer contains a water-soluble cellulose ether and an anionic polymer and prevents adhesion of the solid preparation to the inner wall of the oral cavity. Such an anti-adhesive composition or anti-adhesive layer is dissolved by a small quantity of water or moisture (e.g., saliva) and more certainly forms an aqueous liquid coat around the gel formed from the gel-forming layer due to water absorption and swelling. Accordingly, the direct adhesion (attachment) of the gel-forming layer to the inner wall of the oral cavity can be prevented, and even if part of the gel-forming layer is adhered, the gel-forming layer is easily separated from the inner wall. Moreover, for oral administration, the adhesion of the solid preparation to the inner wall of the oral cavity over a longer period of time can certainly be prevented.

The water-soluble cellulose ether may include an alkyl cellulose [for example, a methyl cellulose (MC)], a hydroxyalkyl cellulose [for example, a hydroxyethyl cellulose (HEC) and a hydroxypropyl cellulose (HPC)], and a hydroxyalkylalkyl cellulose [for example, a hydroxyethylmethyl cellulose (HEMC) and a hydroxypropylmethyl cellulose (HPMC) (e.g., HPMC2208, HPMC2906, and HPMC2910)], a carboxymethyl cellulose [e.g., a carboxymethyl cellulose (CMC) and a CMC-sodium], and others. These cellulose ethers may be used alone or in combination.

Among these water-soluble cellulose ethers, the preferred one includes at least one member selected from the group consisting of a methyl cellulose, a hydroxyethyl cellulose, a hydroxyethylmethyl cellulose, a hydroxypropyl cellulose, and a hydroxypropylmethyl cellulose. Incidentally, for the water-soluble cellulose ether, the alkyl cellulose, the hydroxyalkyl cellulose (e.g., a hydroxyethyl cellulose and a hydroxypropyl cellulose), the hydroxyalkylalkyl cellulose (e.g., a hydroxyC₂₋₃alkylmethyl cellulose such as anHEMC or an HPMC), and the alkyl cellulose (e.g., anMC) seems to have an action preventing the adhesion of the solid preparation to the inner wall of the oral cavity in descending order of degree.

In the hydroxyalkylmethyl cellulose, the content of ether groups derived from all hydroxyl groups of the cellulose is not particularly limited to a specific one. In order to prevent the adhesion of the solid preparation to the inner wall of the oral cavity, it is preferable that the average substitution degree of methyl group be larger and the average substitution degree of hydroxyalkyl group be smaller. Concretely, the methoxy group content (substitution ratio) may for example be about 5 to 40%, preferably about 10 to 35%, and more preferably about 15 to 30%; and the hydroxyalkoxy group content (substitution ratio) may for example be about 0.1 to 20%, preferably about 1 to 15%, and more preferably about 2 to 10%. The ratio of the methoxy group content (substitution ratio) relative to the hydroxyalkoxy group content (substitution ratio) may for example be about 90/10 to 50/50, preferably about 85/15 to 60/40, and more preferably about 80/20 to 70/30, as the methoxy group/the hydroxyalkoxy group.

Among the hydroxyalkylmethyl celluloses, an HPMC is preferred. Representative examples of the HPMC may include HPMC2208, HPMC2906, and HPMC2910, and HPMC2910 is particularly preferred.

The viscosity of the water-soluble cellulose ether for a 2% by mass aqueous solution at 20°C may be not more than 50mPa·s, preferably not more than 40 mPa·s, and more preferably about 1 to 30 mPa·s. Probably or presumably because of more rapid dissolution in a small quantity of water (e.g., saliva) and formation of a lower viscous aqueous liquid coat, a water-soluble cellulose ether having a lower viscosity can effectively prevent the adhesion of the solid preparation to the inner wall of the oral cavity.

The content of the water-soluble cellulose ether of the whole anti-adhesive layer may be selected from the range of about 20 to 99% by mass (e.g., about 30 to 98% by mass) and may usually be about 50 to 95% by mass (e.g., about 60 to 95% by mass) and preferably about 70 to 90% by mass (e.g., about 75 to 90% by mass).

As far as the anionic polymer can be dissolved in water (e.g., saliva) in an environment of the oral cavity, there are no particular limitations thereon. For example, the anionic polymer may include the water-soluble polymer (an anionic polymer such as a carboxy group-containing polymer, a sulfonic acid group-containing polymer, or a phosphoric acid group-containing polymer) as described as the gel-forming agent for the gel-forming layer. The anionic polymer may form, for example, a salt with an inorganic base [e.g., an alkali metal (such as sodium or potassium) and ammonia] or an organic base [e.g., monoethanolamine, diethanolamine, triethanolamine, and dimethylaminoethanol]. The preferred anionic polymer includes the above-mentioned carboxy group-containing polymer, particularly a (meth) acrylic acid-series polymer comprising a (meth)acrylic acid unit as an essential polymerizable component [a homo- or copolymer of (meth)acrylic acid].

The monomer copolymerizable with (meth)acrylic acid may include the copolymerizable monomer described in the gel-forming agent and may be used alone or in combination. For the (meth) acrylic acid-series polymer, the ratio (mass ratio) of the (meth) acrylic acid (or a salt thereof) relative to the copolymerizable monomer is not particularly limited to a specific one as far as the (meth) acrylic acid-series polymer is water-soluble, and for example, the ratio is the same as that described in the gel-forming agent.

As the (meth)acrylic acid-series polymer, there may be mentioned an acrylic acid-series polymer [for example, a polyacrylic acid,an acrylic acid-alkylacrylatecopolymer (e.g., an acrylic acid-methyl acrylate copolymer and an acrylic acid-ethyl acrylate copolymer), and an acrylic acid-alkyl methacrylate copolymer (e.g., acrylic acid-methyl methacrylate and acrylic acid-ethyl methacrylate)],and a methacrylic acid-seriespolymer(e.g., methacrylic acid-alkyl acrylate copolymer such as a methacrylic acid-methyl acrylate copolymer or a methacrylic acid-ethyl acrylate copolymer). These (meth)acrylic acid-series polymers may be used alone or in combination. The viscosity of the anionic polymer for a 0.2% by mass aqueous solution is usually the same as the viscosity of the aqueous solution of the above-mentioned gel-forming agent.

Representative examples of the (meth)acrylic acid-series polymer may include a carboxyvinyl polymer (tradename:CARBOPOL),a poly(sodium acrylate),a partially neutralized product of a polyacrylic acid, a methacrylic acid-n-butyl acrylate copolymer, and a methacrylic acid copolymer LD (trade name: EUDRAGIT L-30D55). Among these (meth)acrylic acid-series polymers, the preferred one includes an acrylic acid-series polymer obtained by using acrylic acid as a main monomer, particularly a carboxyvinyl polymer (e.g., CARBOPOL and HIVISWAKO exemplified in the above-mentioned gel-forming agent).

The anionic polymer content of the anti-adhesive composition may be selected from the range in which the anti-adhesive layer can rapidly absorb water to form a liquid coat while preventing the adhesion of the solid preparation to the inner wall of the oral cavity, and may for example be about 0.1 to 50% by mass (e.g., about 1 to 30% by mass) in terms of a solid content or a non-volatile matter. The anionic polymer content of the whole anti-adhesive composition may be about 1 to 25% by mass (e.g., about 2 to 20% by mass) and preferably about 3 to 17% by mass (e.g., about 5 to 15% by mass) in terms of a non-volatile matter.

Depending on the species of the water-soluble cellulose ether and anionic polymer, when the water-soluble cellulose ether and the anionic polymer are the same species as the base material of the gel-forming layer and the gel-forming agent, respectively, the ratio of the water-soluble cellulose ether relative to the anionic polymer in the anti-adhesive composition is usually larger than the ratio of the base material relative to the gel-forming agent (an anionic polymer such as a carboxyvinyl polymer) of the gel-forming layer. The mass ratio of the water-soluble cellulose ether relative to the anionic polymer in terms of a solid content may be selected from the range of about 99.9/0.1 to 75/25 (e.g., about 99/1 to 80/20), and may usually be about 99.9/0.1 to 85/15 (e.g., about 99/1 to 85/15) and preferably about 95/5 to 85/15 (e.g., about 92/18 to 87/13), as the water-soluble cellulose ether/the anionic polymer. The ratio of the water-soluble cellulose ether relative to 100 parts by mass of the anionic polymer may for example be about 100 to 2000 parts by mass (e.g., about 200 to 1500 parts by mass), preferably about 300 to 1200 parts by mass (e.g., about 500 to 1000 parts by mass), and more preferably about 600 to 900 parts by mass.

The anti-adhesive composition is usually employed in a liquid form (such as a solution or a dispersion). The anti-adhesive layer has a higher or lower viscosity depending on the species of the water-soluble cellulose and that of the anionic polymer, so that the anti-adhesive layer cannot be formed smoothly in some cases. Thus, the anti-adhesive composition may contain a viscosity modifier for adjusting the viscosity, particularly a viscosity reducing agent or an auxiliary. As the viscosity reducing agent, there may be mentioned a water-soluble metal compound or an electrolyte, a water-soluble organic solvent, and others. The water-soluble metal compound or the electrolyte may include, for example, an alkali metal salt [for example, an inorganic acid salt (e.g., a chloride such as sodium chloride or potassium chloride; a carbonate such as sodium carbonate; and a phosphate such as sodium monohydrogenphosphate or sodium dihydrogenphosphate) and an organic acid salt (e.g., sodium acetate, sodium lactate, and sodium citrate)], an alkaline earth metal salt [for example, an inorganic acid salt (a chloride such as magnesium chloride or calcium chloride; a sulfate such as calcium sulfate; a phosphate such as calcium hydrogenphosphate; and a silicate such as magnesium silicate) and an organic acid salt (e.g., calcium acetate and calcium lactate)], and a tri- to polyvalent metal salt [forexample, an inorganic acid salt (e.g., a chloride such as aluminum chloride; a sulfate such as aluminum sulfate; a phosphate such as aluminum phosphate; and a silicate such as aluminum silicate) and an organic acid salt (e.g., aluminum acetate)]. Examples of the water-soluble organic solvent may include an alcohol such as ethanol or ethylene glycol, a ketone such as acetone, a cyclic ether such as dioxane, a cellosolve such as a methyl cellosolve, and N-methyl-2-pyrrolidone. These viscosity reducing agents may be used alone or in combination.

Among these components, a metal salt highly reducing the viscosity of the solution (for example, an alkali metal salt and an alkaline earth metal salt) is used practically. The amount of the viscosity reducing agent relative to 100 parts by mass of the total amount of the water-soluble cellulose ether and the anionic polymer may for example be selected from the range of about 0 to 200 parts by mass and may usually be about 1 to 100 parts by mass, preferably about 5 to 50 parts by mass, and more preferably about 10 to 30 parts by mass.

Incidentally, the polyvalent metal salt (an alkaline earth metal salt, a tri- to polyvalent metal salt) may function as a crosslinking agent for the anionic polymer. When such a polyvalent metal salt is used for the anti-adhesive composition, the amount of the polyvalent metal salt is smaller than the amount of the crosslinking agent relative to 100 parts by mass of the total amount of the base material and the gel-forming agent in the gel-forming layer. The amount of the polyvalent metal salt in the anti-adhesive composition may for example be about 0 to 2 parts by mass (e.g., about 0.01 to 1.5 parts by mass), preferably about 0. 05 to 1 parts by mass, and more preferably about 0.1 to 0.5 parts by mass (e.g., about 0.2 to 0.4 parts by mass) relative to 100 parts by mass of the total amount of the water-soluble cellulose ether and the anionic polymer (e.g., a carboxyvinyl polymer). Incidentally, the ratio of the polyvalent metal salt relative to 100 parts by mass of the anionic polymer (e.g., a carboxyvinyl polymer) may for example be about 0.1 to 10 parts by mass (e.g., about 0.5 to 7.5 parts by mass), preferably about 1 to 5 parts by mass, and more preferably about 1.5 to 3.5 parts by mass (e.g., about 2 to 3 parts by mass).

The anti-adhesive composition of the present invention may contain the various additives as described above, such as the water absorption promoter (for example, glycerin), the masking agent for masking the taste or smell of the active ingredient, the plasticizer (for example, glycerin triacetate, diethyl phthalate, and triethyl citrate), the antiseptic agent or the preservative (for example, methyl hydroxybenzoate, propyl hydroxybenzoate, sodium edetate, potassium sorbate, and sodium dehydroacetate), the antioxidant (such as ascorbic acid or tocopherol acetate), and the coloring agent (for example, titanium oxide, and edible lake coloring agent). The masking agent may include an acidifier or an acidulant (e.g., citric acid, tartaric acid, and fumaric acid), a sweetening agent (e.g., saccharin, glycyrrhizinic acid, aspartame, stevioside, acesulfame potassium, and a saccharide), an algefacient (e.g., menthol, mentha oil, peppermint, and spearmint), a natural or synthetic flavoring agent (or perfume), and others. Among these masking agents, a saccharide (a sugar such as lactose, white sugar or refined sugar, glucose, or sucrose, a sugar alcohol such as mannitol, sorbitol, or xylitol) is preferred.

These components may also be used alone or in combination. The amount of these components may be not more than 20 parts by mass (e.g., about 0.01 to 15 parts by mass, preferably about 0.05 to 10 parts by mass, and more preferably about 0.1 to 10 parts by mass) relative to 100 parts by mass of the total amount of the water-soluble cellulose ether and the anionic polymer (in terms of a solid content) .

It is sufficient that the anti-adhesive layer covers at least part of the surface of the gel-forming layer (for example, not less than 50% of the surface area of the gel-forming layer (e.g., about 50 to 100%, preferably about 87 to 100%, and more preferably about 90 to 100%)). The anti-adhesive layer practically covers the whole of the gel-forming layer or at least upper and under surfaces thereof. The anti-adhesive layer may cover the surface of the gel-forming layer uniformly or nonuniformly (e.g., scatteringly in a polygonal pattern (e.g., quadrilateral pattern), a circular pattern, or a grid pattern).

In order to easily permeate even a small quantity of water (such as saliva) into the anti-adhesive layer, the thickness of the anti-adhesive layer may be not more than 50 µm (e.g., about 1 to 50 µm, preferably about 5 to 45 µm, and more preferably about 10 to 40 µm).

The total thickness of the gel-forming layer and the anti-adhesive layer may for example be about 5 to 1000 µm, preferably about 10 to 500 µm (e.g., about 15 to 250 µm), and more preferably about 20 to 100 µm (e.g., about 25 to 75 µm). Moreover, the thickness ratio of the gel-forming layer relative to the anti-adhesive layer may be selected from the range of about 5/95 to 95/5 (e.g., about 10/90 to 90/10) and may be about 15/85 to 50/50 and more preferably about 20/80 to 40/60 (e.g., about 20/80 to 30/70), as the gel-forming layer/the anti-adhesive layer. By controlling the thickness ratio of the gel-forming layer relative to the anti-adhesive layer, the gel-forming layer rapidly absorbs water through the anti-adhesive layer and swells to form a gel layer having a significantly improved slipperiness in a short period of time, and the anti-adhesive layer can form an aqueous liquid coat as the surface layer. Probably due to such a structure, the solid preparation (solid preparation for oral administration) can easily be swallowed without adhesion to the inner wall of the oral cavity even in absence of water and improve the ease (or easiness) of taking the preparation significantly.

### [Shape of solid preparation]

It is sufficient that the solid preparation comprises the drug-containing unit, the gel-forming layer, and the anti-adhesive layer, and the adhesive layer is not necessarily required. Moreover, if necessary, an enteric coating layer, a gastric-soluble coating layer, a water-insoluble coating layer, or other layers may be formed at an appropriate interlayer of the drug-containing unit, the gel-forming layer, and the anti-adhesive layer. The enteric component may include, for example, an enteric base material described in the above-mentioned drug-containing unit. The gastric-soluble component may include, for example, a gastric-soluble base material described in the above-mentioned drug-containing unit. As the water-insolublecomponent,forexample,theremay mentioned an ethyl cellulose, an ethyl acrylate-methyl methacrylate copolymer, and a lipid.

The solid preparation (or solid preparation for oral administration) of the present invention may be in the form corresponding to the drug-containing unit or in the form in which the gel-forming layer and the anti-adhesive layer are extended from the periphery of the drug-containing unit. Moreover, the solid preparation of the present invention may be a film-covered (or laminate) preparation in the form of a flat shape or a discoid shape, for example, a flat or discoid preparation having the drug-containing unit enclosed (or wrapped) with a film- or sheet-like covering layer(s). The plane shape of the film-covered preparation may for example be a polygon (e.g., a quadrilateral), a circle, and an ellipse. According to the solid preparation of the present invention, the gel-forming layer and the anti-adhesive layer improves the slipperiness in the oral cavity by even a small quantity of water. Therefore, even when the film-covered preparation has a large flat-surface area, the preparation can easily be swallowed. The area of the flat surface of the film-covered preparation is not particularly limited to a specific one, and may be about 0.01 to 10 cm (e.g., about 0.05 to 9 cm², preferably about 0.1 to 8 cm², and about more preferably 0.5 to 7 cm²).

Incidentally, the surface of the solid preparation may be embossed, if necessary. Moreover, if necessary, the surface of the solid preparation may be sugar-coated.

### [Process for producing solid preparation]

The solid preparation of the present invention may be prepared by applying the anti-adhesive composition (coating agent) to the gel-forming layer covering the drug-containing unit to cover the drug-containing unit with the gel-forming layer. The drug-containing unit can be prepared using the active ingredient and the additive according to a conventional manner (such as granulation or tableting), as described above. Moreover, each layer of the solid preparation can be produced by each applying a coating composition corresponding to each layer to the drug-containing unit sequentially. Each of the coating compositions (e.g., anti-adhesive composition or coating agent) corresponding to each layer can be prepared by dispersing or dissolving constituents of each layer (for example, the anti-adhesive layer) in a liquid medium such as water (e.g., a purified water) or a lower alcohol (e.g., ethanol), optionally an organic solvent. Incidentally, if necessary, the resulting coating composition (liquid coating composition or coating agent) may be defoamed.

Depending on the dosage form, a method for coating the drug-containing unit with the coating composition may include, for example, a pan coating, a fluidizedbed coating, a tumbling coating, and a tumbling fluidized bed coating. For example, coating (applying), spraying, and impregnation or dipping may be used for coating the drug-containing unit with the coating composition. Incidentally, each coating composition may be coated successively after drying or without drying.

For the preparation of the solid preparation of the present invention, there may be used lamination or stacking of each layer to the drug-containing unit by flow-casting, coating (applying), or other means. For example, the solid preparation of the present invention may be prepared by a process which comprises a step for applying the anti-adhesive composition (coating agent) to a releasable (separable) substrate to form an anti-adhesive layer (an anti-adhesive layer forming step), a step for laminating a gel-forming layer on the anti-adhesive layer (a gel-forming layer laminating step), and an optional step for laminating an adhesive layer on the gel-forming layer (an adhesive layer laminating step), and a step for interposing a drug-containing unit between two laminates prepared through these steps and adhering (or bonding) these laminates (an adhering step).

The releasable substrate is not particularly limited to a specific one, and, for example, a glass plate, a plastic film, and a release sheet maybe used. If necessary, these releasable substrates may be embossed by a conventional manner.

The anti-adhesive layer, the gel-forming layer, and the adhesive layer can be formed by coating the releasable substrate with each liquid coating composition using a conventional film-forming method (for example, a method using coating (applying) such as flow-casting, or spraying) . Incidentally, the anti-adhesive layer is not essentially formed on the whole surface of the gel-forming layer. In order to form the aqueous liquid coat and the gel layer uniformly and improve the ease of swallowing the preparation, the whole surface of the gel-forming layer is practically coated with the anti-adhesive layer. Moreover, the adhesive layer may be formed by coating the gel-forming layer partly.

In the adhering step, a pair of laminates can be adhered (bonded) to each other while interposing the drug-containing unit between these laminates with the gel-forming layers (or adhesive layers) facing each other. The drug-containing unit can be arranged at a predetermined position with the use of a method for positioning the preparation at a predetermined site (or area), coating (applying), spraying, dropping, ink-jetting, screen-printing, or others. Incidentally, when an embossed releasable substrate having the gel-forming layer (or adhesive layer) is used, the drug-containing unit may be placed in a recessed area formed in the gel-forming layer (or adhesive layer).

As a method for adhering the laminates, for example, thermal adhesion (or hot-melting) or other means can be utilized when a heat (or thermal) adhesive is used. The temperature of the thermal adhesion may for example be about 70 to 150°C (e.g., about 75 to 140°C, preferably about 80°C to 130°C, and more preferably about 85 to 120°C).

The solid preparation can be produced by adhering the periphery of the drug-containing unit to prepare a laminate (or laminated product) having the above layers, and then punching out the periphery of the drug-containing unit in a predetermined shape (e.g., a circular shape, an elliptical shape, and a polygonal shape) depending on the shape of the drug-containing unit.

### EXAMPLES

Hereinafter, the following examples are intended to describe this invention in further detail and should by no means be interpreted as defining the scope of the invention.

### (Example 1)

### (a) Step for forming anti-adhesive layer

To 380 parts by mass of purified water, 0.27 parts by mass of calcium chloride (Calcium chloride H, manufactured by Tomita Pharmaceutical Co., Ltd.) as a viscosity reducing agent was added and dissolved by stirring. To this solution was slowly added 10.0 parts by mass of a carboxyvinyl polymer (a polyacrylic acid, CARBOPOL 974P, manufactured by Noveon, viscosity of 0.2% by mass aqueous solution (20°C) : 12100 mPa·s) with stirring, and the mixture was stirred for one hour. The mixture containing each component was heated to 80°C. To the mixture was slowly added 81.63 parts by mass of a hydroxypropylmethyl cellulose (TC-5E, manufactured by Shin-Etsu Chemical Co., Ltd., viscosity of 2% by mass aqueous solution (20°C) : 3 mPa·s) with stirring, the resulting mixture was stirred for 15 minutes, and the temperature of the mixture was decreased to 30°C, and the mixture was stirred for one hour. To the resulting mixture was added 8.1 parts by mass of glycerin (Japanese Pharmacopoeia, concentrated glycerin, manufactured by Asahi Denka Kogyo K.K.) as a water absorption promoter, and the mixture was stirred for 15 minutes to give a liquid coating composition A (anti-adhesive composition) .

The liquid coating compositionAwas fully defoamed. A poly(ethylene terephthalate) film (SP-PET3811, manufactured by LINTEC Corporation), as a releasable substrate, had a releasably treated surface. The liquid coating composition A was spread-coated (spread-applied) on an untreated surface of the film using an applicator with an adjusted gap and dried at 80°C for 10 minutes to form an anti-adhesive layer having a thickness of 28 µm after drying, and a laminate intermediate "a" (a laminate of the anti-adhesive layer/the releasable substrate) was obtained.

### (b) Step for laminating gel-forming layer

To 700 parts by mass of purified water, 0.6 parts by mass of calcium chloride (Calcium chloride H, manufactured by Tomita Pharmaceutical Co., Ltd.) as a crosslinking agent was added and dissolved by stirring for 5 minutes. To this solution was slowly added 22.7 parts by mass of a polyacrylic acid (CARBOPOL 974P, manufactured by Noveon, viscosity of 0.2% by mass aqueous solution (20°C) : 12100 mPa·s) with stirring, and the mixture was stirred for one hour. To the mixture was slowly added 68.6 parts by mass of a poly(vinyl alcohol) (GOHSENOL EG05T, manufactured by The Nippon Synthetic Chemical Industry Co ., Ltd.) with stirring. After the mixture was stirred for 15 minutes, the mixture containing each component was heated to 80°C and stirred for one hour. Thereafter, the mixture containing each component was cooled to 30°C . To the mixture was added 8.1 parts by mass of glycerin (Japanese Pharmacopoeia, concentrated glycerin, manufactured by Asahi Denka Kogyo K.K.) as a water absorption promoter, and the resulting mixture was stirred for about 15 minutes to give a liquid coating composition B.

The liquid coating composition B was fully defoamed. The liquid coating composition B was spread-coated (spread-applied) on the anti-adhesive layer formed in the step (a) using an applicator with an adjusted gap and dried at 80°C for 5 minutes to give a laminate intermediate "b" (a laminate of the gel-forming layer/the anti-adhesive layer/the releasable substrate) having a gel-forming layer of 9 µm thickness after drying.

### (c) Step for laminating adhesive layer

To 220 parts by mass of water, 19.4 parts by mass of glycerin (Japanese Pharmacopoeia, concentrated glycerin, manufactured by Asahi Denka Kogyo K.K.) as a plasticizer was slowly added with stirring and dissolved. Thereafter, to the solution was slowly added 80.6 parts by mass of a polyvinylpyrrolidone (PVP K-90, manufactured by ISP Japan Ltd.) as a base material with stirring. The mixture was stirred for 60 minutes to give a liquid coating composition C-1.

The liquid coating composition C-1 was fully defoamed. The liquid coating composition C-1 was spread-coated (spread-applied) on the gel-forming layer formed in the step (b) using an applicator with an adjusted gap and dried at 80°C for 10 minutes to give a laminate intermediate "c" (a laminate of the adhesive layer/the gel-forming layer/the anti-adhesive layer/the releasable substrate) having an adhesive layer (intermediate layer) of 93 µm thickness after drying.

### (d) Adhering step

Two laminates (first and second laminates), each prepared by such a process, had a structure of the adhesive layer/the gel-forming layer/the anti-adhesive layer/the releasable substrate. A placebo tablet (diameter of 8 mm, thickness of 2.6 mm) was placed on the adhesive layer of the first laminate, and then the adhesive layer of the second laminate was superposed on the tablet to prepare an intermediate having a structure of the releasable substrate/the anti-adhesive layer/the gel-forming layer/the adhesive layer/the drug-containing unit/the adhesive layer/the gel-forming layer/the anti-adhesive layer/the releasable substrate. In order to cover the drug-containing unit, the adhesive layers were bonded to each other at 100°C under 1 kgf/cm² for 1 second. Then, the releasable substrates were removed from the anti-adhesive layers to prepare a laminate having the anti-adhesive layer, the gel-forming layer, the adhesive layer, and the drug-containing unit. A circular shape having a diameter of 15 mm was punched out of the periphery of the drug-containing unit of the laminate for a secondary processing to give a solid preparation.

### Example 2

In the same manner as in Example 1 except for using a methyl cellulose (METOLOSE SM-4, manufactured by Shin-Etsu Chemical Co., Ltd., viscosity of 2% by mass aqueous solution (20°C): 4.2 mPa·s) instead of the hydroxypropylmethyl cellulose used for the anti-adhesive composition, a solid preparation was produced.

### Comparative Example 1

In the same manner as in Example 1 except that the anti-adhesive layer was not formed, a solid preparation was prepared.

### Test Examples

For each solid preparation obtained in Examples and Comparative Examples, the administration test of the solid preparation was performed for four subjects. The adherability of the solid preparation to the inner wall of the oral cavity and the ease of swallowing the solid preparation were evaluated as follows.

### [Adherability evaluation]

The oral cavity of a subj ect was washed by gargling. After 2 minutes, the solid preparation was so put into the oral cavity without water as to be adhered to the palate (or the upper wall of the oral cavity) easily. Whether or not the solid preparation was adhered to the palate was examined. When the solid preparation was adhered to the palate, whether or not the solid preparation was separable from the palate was examined and evaluated.

### [Evaluation of swallowing (evaluation of ease of taking preparation)]

The oral cavity of a subj ect was washed by gargling. After 2 minutes, the oral administration preparation was put into the oral cavity without water, and swallowed. The degree of swallowing the preparation was evaluated.

The results showed that the solid preparation of Comparative Example 1 was adhered to the oral cavity for all subjects, and that two of the four subjects could not take the preparation because the preparation could not be separated from the oral cavity. On the other hand, for the solid preparation of Example 1, the adhesion of the preparation to the oral cavity was not found for three subjects, and the preparation was easily separated from the oral cavity for the remaining one subject. Moreover, the adhesion of the solid preparation of Example 2 to the oral cavity was not found for all subjects.

In addition, each of the solid preparations of Examples 1 and 2 could be administered smoothly without uncomfortable feeling compared with the preparation of Comparative Example 1 and had an improved comfortability of taking the preparation.

### INDUSTRIAL APPLICABILITY

The solid preparation of the present invention effectively prevents the adhesion of the preparation to the inner wall of the oral cavity, and significantly improves the comfortability of taking the preparation. In particular, the solid preparation can be swallowed with a small quantity of water (e. g. , saliva). Thus, the present invention can provide a preparation having extensively improved medication compliance from infants (babies and little children) to elderly people who cannot swallow the conventional solid preparation easily.

### DESCRIPTION OF REFERENCE NUMERALS

1 ··· Solid preparation
2 ··· Drug-containing unit
3 ··· Adhesive layer
3a ··· First adhesive layer
3b ··· Second adhesive layer
4 ··· Gel-forming layer
4a ··· First gel-forming layer
4b ··· Second gel-forming layer
5 ··· Anti-adhesive layer
5a ··· First anti-adhesive layer
5b ··· Second anti-adhesive layer

## Claims

1. An anti-adhesive composition for preventing adhesion to an inner wall of an oral cavity, which is applied to a gel-forming layer covering a drug-containing unit and contains a water-soluble cellulose ether and an anionic polymer.

2. An anti-adhesive composition according to Claim 1, wherein the water-soluble cellulose ether have a viscosity of not more than 50 mPa·s for a 2% by mass aqueous solution at 20°C.

3. An anti-adhesive composition according to Claim 1 or 2, wherein the water-soluble cellulose ether comprises at least one member selected from the group consisting of a methyl cellulose, a hydroxyethyl cellulose, a hydroxyethylmethyl cellulose, a hydroxypropyl cellulose, and a hydroxypropylmethyl cellulose.

4. An anti-adhesive composition according to any one of Claims 1 to 3, wherein the anionic polymer comprises a homo- or copolymer of (meth)acrylic acid.

5. An anti-adhesive composition according to any one of Claims 1 to 4, which covers the gel-forming layer formed with a composition containing a gel-forming agent, a base material, and a crosslinking agent, and the ratio of the water-soluble cellulose ether relative to the anionic polymer is larger than the ratio of the base material relative to the gel-forming agent.

6. An anti-adhesive composition according to any one of Claims 1 to 5, wherein the mass ratio of the water-soluble cellulose ether relative to the anionic polymer is 99. 9/0.1 to 85/15, as a ratio of the water-soluble cellulose ether/the anionic polymer, in terms of a solid content .

7. An solid preparation comprising
a drug-containing unit,
a gel-forming layer for covering the drug-containing unit, and
an anti-adhesive layer for covering the gel-forming layer, wherein the anti-adhesive layer is formed with an anti-adhesive composition recited in any one of Claims 1 to 6.

8. A solid preparation according to Claim 7, wherein the total thickness of the gel-forming layer and the anti-adhesive layer is 5 to 1000 µm, and the ratio of the thickness of the gel-forming layer relative to the thickness of the anti-adhesive layer is 15/85 to 50/50 as a ratio of the gel-forming layer/the anti-adhesive layer.

9. A solid preparation according to Claim 7 or 8, which is a film-covered preparation.

10. A process for producing a solidpreparation which prevents adhesion of the solid preparation to an inner wall of an oral cavity, the process comprising
applying an anti-adhesive composition recited in any one of Claims 1 to 6 to a gel-forming layer covering a drug-containing unit.
